**Europäisches Patentamt**

**European Patent Office**

**Office européen des brevets**

(19)

(11) Numéro de publication: **0 147 261**
**B1**

(12) **FASCICULE DE BREVET EUROPEEN**

(45) Date de publication du fascicule du brevet: **18.07.90**

(51) Int. Cl.⁵: **A 61 K 37/12**

(21) Numéro de dépôt: **84402242.6**

(22) Date de dépôt: **07.11.84**

(54) **Médicament et composition médicamenteuse pour le traitment et/ou la prevention des maladies de la peau mettant en jeu un processus inflammatoire.**

(30) Priorité: **09.11.83 LU 85081**

(43) Date de publication de la demande:
**03.07.85 Bulletin 85/27**

(45) Mention de la délivrance du brevet:
**18.07.90 Bulletin 90/29**

(84) Etats contractants désignés:
**BE CH DE FR GB IT LI NL SE**

(56) Documents cités:
**CH-A- 188 317**

**CHEMICAL ABSTRACTS, vol. 96, no. 9, 1 mars 1982, page 260, résumé no. 64948e, Columbus, Ohio, US; K. YOSHINO et al.: "Antioxidation effects of human epidermal keratin on skin surface lipid peroxides", & NIPPON HIFUKA GAKKAI ZASSHI 1981, 91(11), 1175-7. Rapport d'essai, page 35 du dossier**

(73) Titulaire: **L'OREAL**
**14, Rue Royale**
**F-75008 Paris (FR)**

(72) Inventeur: **Brod, Joel**
**89, rue de l'Ourcq**
**F-75019 Paris (FR)**
Inventeur: **Kermici, Michel**
**36, rue de Picpus**
**F-75012 Paris (FR)**

(74) Mandataire: **Peuscet, Jacques**
**Cabinet Peuscet 68, rue d'Hauteville**
**F-75010 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# EP 0 147 261 B1

## Description

La présente invention se rapporte à un médicament, ainsi qu'à une composition médicamenteuse, permettant notamment de traiter ou de prévenir, par application topique, les maladies de la peau mettant en jeu un processus inflammatoire, comme les érythèmes par irradiation, les photodermatoses, l'épidermolyse, l'acanthose, l'hyperplasie, les inflammations se produisant dans le processus de la carcinogénèse, les informations des comédons dans l'acné et les dermatoses séborrhéiques.

On a découvert récemment, ce qui est illustré par une abondante littérature, que les compositions qualitatives des lipides cutanés de surface secrétés par la peau varient entre un sujet présentant une peau saine et un sujet présentant une maladie ou une tendance à une maladie impliquant un processus inflammatoire, par exemple, l'une des maladies énumérées ci-dessus. En particulier, dans les sécrétions lipidiques cutanées se trouvant dans les glandes sébacées et à l'intérieur d'un comédon ouvert ou fermé, on a découvert la présence d'une sous-classe de produits lipidiques, qui sont constitués par les lipides cutanés (essentiellement le squalène, l'acide linoléique et l'acide arachidonique) à l'état (per)oxydé. Ces lipides (per)oxydés et leurs produits de dégradation sont irritants pour la peau et peuvent entraîner de sérieux dommages par processus inflammatoire, s'ils sont formés en grande quantité.

On sait par ailleurs, qu'en dehors des lipides cutanés précités qui proviennent du tégument, il peut se trouver, sur la peau, des lipides ou, en général, des liposolubles apportés par un traitement. Une désignation des "liposolubles" est fournie notamment dans la demande de brevet européen publiée sous le n° 00 99 780, les "liposolubles" désignant de façon générale:

a) des composés insolubles dans l'eau mais solubles dans des solvants polaires ou non-polaires, pris isolément ou en mélange; dans cette catégorie se trouvent notamment les lipides tels que définis par la classification de LEHNINGER (LEHNINGER—Editions Flammarion—2ème édition, Chapitre 11, pages 275—303);

b) des composés solubles dans un ou plusieurs lipides, seuls ou solubilisés dans un ou plusieurs lipides.

Dans ce qui suit, on désignera par "lipides présents sur la peau", aussi bien les lipides qui proviennent du tégument que les liposolubles qui peuvent avoir été apportés par un traitement.

La société déposante à découvert une classe de composés qui, d'une part, ont une très haute affinité pour les (per)oxydes lipidiques présents sur la peau, bien supérieure à celle qu'ils ont pour les mêmes lipides à l'état non (per)oxydé; ces composés forment, avec les lipides (per)oxydés, des produits complexes, avec interruption du mécanisme de propagation des radicaux qui intervient dans la formation des lipides (per)oxydés; ces composés préviennent, en outre, la formation des (per)oxydes de lipides dans les conditions de (per)oxydation par exposition au rayonnement ultra-violet (A+B), et préviennent ainsi l'apparition d'érythèmes provoqués par cette exposition.

Ce double effet de "pompage sélectif" des lipides (per)oxydés et de protection vis-à-vis de l'oxydation des lipides considérés rend cette classe de composés particulièrement intéressante dans le traitement de maladies inflammatoires de la peau dues aux lipides présents sur la peau à l'état (per)oxydé, du fait que leur application sur la peau permet, en premier lieu, de fixer les lipides déjà (per)oxydés en donnant des complexes qu'il sera aisé d'éliminer en vue de désintoxiquer le substrat cutané, et, en second lieu, d'empêcher la (per)oxydation des lipides non encore (per)oxydés. On soulignera en outre le rôle protecteur de cette classe de composés vis-à-vis de l'apparition de l'érythème sous rayonnement UV: on a observé qu'une peau exposée au soleil traitée par un composé de cette classe ne présente pas d'érythème; alors qu'une peau sujette à l'apparition d'érythèmes, non protégée, sera atteinte. Par ailleurs, lorsque l'érythème est apparau sans que l'on ait protégé la peau au préalable, l'application des composés précités joue un rôle calmant par piégeage des produits (per)oxydés formés qui sont irritants pour la peau. Concernant les liposolubles apportés par un traitement, par exemple la vitamine A acide, on sait en effet qu'ils peuvent être source d'érythèmes, lorsque le sujet traité s'expose au soleil. On voit bien le rôle protecteur que peuvent jouer dans ces cas-là les composés précités, qui, pà également, préviennent et/ou le cas échéant, empêchent d'aggraver l'érythème solaire.

Cette classe de composés consiste en des "kératines délipidées", telles qu'elles sont décrites dans la demande de brevet européen publiée sous le n° EP—A—00 99 780.

La présente invention a donc pour objet le médicament, utile notamment par son effet complexant sélectif vis-à-vis des lipides présents sur la peau à l'état (per)oxydé et/ou par son effet protecteur vis-à-vis de la (per)oxydation des lipides présents sur la peau et/ou par son effet préventif d'érythèmes et permettant, en particulier, de traiter et/ou de prévenir par application topique, les maladies de la peau mettant en jeu un processus inflammatoire, caractérisé par le fait qu'il consiste en au moins un polymère kératinique provenant d'une matière première contenant au moins une kératine d'origine animale, ledit polymère étant au moins partiellement délipidé.

On préfère que le polymère kératinique soit totalement délipidé.

Par ailleurs, la matière première d'origine animale utilisée peut avoir subi une hydrolyse, soit avant délipidation, soit après délipidation.

De préférence, la matière première animale, d'où proviennent les chaînes kératiniques du polymère, est choisie dans le groupe formé par la corne de sabot, notamment de cheval, les museaux et naseaux, notamment de bovins, le peau animale, notamment de porc, les cheveux et les plumes.

2

**EP 0 147 261 B1**

On soulignera que les polymères kératiniques précités sont des polymères à l'état libre, les chaînes kératiniques n'étant pas englobées dans des cellules fermées, comme c'est le cas notamment dans les produits d'origine kératinique ayant subi une délipidation préalable, tels que ceux qui sont définis dans le brevet allemand DE—A 556 488 et dans les brevets des Etats-Unis d'Amérique US—A 3 033 755 et US—A—3 660 566, où les traitements du matériau laissent subsister les cellules, les chaînes polymériques kératiniques, qui constituent les filaments kératiniques intra-cellulaires, n'étant pas libérées. Par ailleurs, les chaînes kératiniques constitutives du polymère kératinique utilisé selon la présente invention sont, dans ce polymère, à une concentration beaucoup plus forte que dans les produits cellulaires de l'état de la technique précité, qui contiennent les ciments extra-cellulaires, les protéines non kératiniques intra-cellulaires et les membranes cellulaires.

Pour obtenir les polymères kératiniques utilisables dans la présente invention, on dispose notamment de deux procédés.

Selon un premier procédé, on réalise une délipidation initiale, totale ou partielle, en soumettant la matière première, sous agitation, pendant un temps compris entre 20 minutes et 24 heures (ce temps dépendant de l'importance souhaitée pour la délipidation) à l'action d'un solvant pour les lipides qui s'y trouvent. De préférence, le solvant, qui permet la délipidation, est un mélange chloroforme/méthanol dans des proportions comprises entre 1/1 et 2/1 en volume, ou encore un mélange dichlorométhane/méthanol dans une proportion 3/1 en volume; on peut, avantageusement, prévoir que la matière première traitée par le solvant soit initialement broyée. Pour obtenir un polymère kératinique à partir de ce matériau, on réalise ensuite une purification de type connu par la succession d'étapes suivantes:

a) on lave l'extrait avec un tampon approprié;
b) on solubilise l'extrait lavé à un pH comprise entre 8,5 et 9,5 dans un milieu solubilisant approprié;
c) on élimine les fractions insolubles et on dialyse la solution;
d) on précipite la kératine en amenant le pH entre 5 et 5,8;
e) éventuellement, on répète plusieurs fois l'ensemble des étapes b), c) et d), ci-dessus;
f) on récupère la kératine précipitée dans la phase d).

Si la purification est suffisante, on obtient ainsi un polymère kératinique totalement hydrosoluble.

On peut, avantageusement, prévoir d'utiliser pour l'étape a) ci-dessus mentionnée un tampon à forte concentration de chlorure de potassium; le milieu solubilisant de l'étape b) est avantageusement, une solution aqueuse ("T.U.M.E.") de tri-hydroxyméthylaminométhane formant un tampon à un pH de 8,9 et contenant de l'urée (8 M) et du mercapto-éthanol (0,2 M).

Selon un deuxième procédé, la matière première kératinique est d'abord soumise, avant tout traitement affectant son chargement lipidique initial, à une extraction permettant d'obtenir une kératine native, notamment par la succession d'étapes a) à f) ci-dessus mentionnée. Après quoi, on délipide, totalement ou partiellement, la kératine native obtenue par un traitement en milieu solvant analogue à celui utilisé pour la délipidation dans le premier procédé.

Les polymères kératiniques utilisés conformément à la présente invention ne sont absolument pas toxiques pour la peau par laquelle ils sont très bien tolérés; ceci tient vraisemblablement au fait qu'ils comportent des chaînes kératiniques d'origine naturelle obtenues à partir d'une matière première d'origine animale.

On a constaté que l'on obtenait des résultats particulièrement intéressants en utilisant, comme matières premières pour les polymères kératiniques selon l'invention, des matériaux kératiniques très solubles dans les tampons alcalins contenant de l'urée, ce qui est le cas des kératines particulières énumérées ci-dessus.

La Société déposante a mis en évidence l'affinité supérieure de kératines délipidées pour la forme peroxydée de lipides cutanés, ainsi que leur effet inhibiteur de la peroxydation de lipides cutanés.

A) Mise en evidence de l'effet de "pompage selectif" de lipides peroxydés
a) préparation d'une kératine délipidée et son chargement avec un lipide cutané choisi

On prépare un polymère kératinique délipidé selon l'un ou l'autre des procédés indiqués ci-dessus et on procède ensuite à son rechargement en un lipide cutané choisi.

Si l'on a utilisé le premier procédé précité pour la préparation du polymère kératinique délipidé, la phase de chargement en lipide peut être effectuée en mettant en contact, sous agitation et pendant un temps compris entre 30 minutes et 12 heures, selon la valeur que l'on veut donner au taux de chargement, le matériau à charger en lipide, mis sous forme particulaire, avant (ou après) purification et extraction du polymère kératinique, avec une solution du lipide de chargement choisi dans un solvant, puis en éliminant par évaporation ledit solvant. L'excès du lipide de chargement peut être éliminé par lavages successifs répétés avec une solution de chlorure de sodium à 9%; dans le cas où le matériau chargé a subi une phase de purification et d'extraction du polymère kératinique, on préfère enlever l'excès de lipide par une solution aqueuse "T.U.M.E." ci-dessus définie.

Si, pour préparer le polymère kératinique délipidé, on a utilisé le second procédé défini ci-dessus, pour recharger la kératine en lipide, on apporte le lipide en question en milieu solvant, on forme le polymère kératinique rechargé au cours d'une phase de solubilisation de la kératine et on précipite ce polymère par variation du pH. Le polymère est alors recueilli par centrifugation; l'excès de lipide est éliminé avec le surnageant. Le culot peut être utilisé tel que (forme solide) ou resolubilisé et redialysé (forme solubilisée);

3

l'association du matériau kératinique avec le lipide est réalisée dans le mélange ("T.U.M.E.") précédemment défini.

On constate que la dérivé obtenu est un complexe formé grâce à une déstabilisation des α-hélices de la kératine suivie d'une ré-aggrégation; ce complexe est caractérisé par une constante d'affinité analogue à celle définie dans le modèle mathématique de Scatchard.

Exemple de préparation:
Première étape: obtention d'une matière première kératinique:

De la peau de porc, préalablement lavée à l'eau, est débarrassée de sont tissu adipeux par grattage. Des carrés de peau de 10 cm×10 cm sont placés dans une enceinte saturée de vapeur d'ammoniac pendant 30 mn à température ambiante. Ces carrés sont ensuite incubés à 37°C pendant une heure dans une solution de trypsine à 0,05% en milieu tampon trihydroxyméthylaminométhane/acide chlorhydrique 5×10⁻² M (pH=7,9). Le stratum cornéum est récolté sous forme de feuillets, comme décrit dans l'article de FERGUSSON, Brit. J. Dermatol. *96*, 21, 1977. Ces feuillets de stratum cornéum sont réduits en poudre par broyage dans l'azote liquide.

Deuxième étape: phase de délipidation:

On solubilise 15 g de stratum cornéum de porc dans 300 ml d'un mélange T.U.M.E. contenant:
—urée: 6 M
—mercapto/éthanol: 0,2 M
—tampon tri-hydroxyméthylaminométhane/acide chlorhydrique: 0,5 M (pH=9).

Cette solubilisation s'effectue sous forte agitation à la température ambiante pendant 24 heures. On centrifuge afin d'éliminer la partie non digérée. On dialyse le surnageant contre un tampon à pH 9.

Après précipitation sélective par de l'acide chlorhydrique à pH 5,5, on recueille le culot de kératine par centrifugation et on procède à la délipidation des kératines à l'aide de 200 ml de chloroforme/méthanol 2/1 v/v. On obtient ainsi des kératines délipidées.

Troisième étape: phase de chargement en lipide:

Les kératines obtenues à la fin de la deuxième étape sont réparties en plusieurs lots de 100 mg et sont re-rechargées soit avec des taux de chargement variant de 1 à 35% d'un lipide cutané peroxydable, mais non peroxydé, soit avec des taux de chargement variant de 1 à 35% du même lipide, mais sous sa forme peroxydée, laquelle est obtenue par exposition sous une lampe "OSRAM ultra-vitalux"® de 300 W, sous agitation en milieu chloroforme pendant 3 heures.

Après évaporation des solvants, on resolubilise dans le milieu T.U.M.E. les kératines chargées; on les précipite par de l'acide chlorhydrique à pH 5,5 et on centrifuge; l'excès de charge est ainsi éliminé avec le surnageant. On procède sur le culot à l'aide d'un mélange chloroforme/méthanol, 2/1, v/v, sous agitation et pendant 12 heures, à l'extraction des substances lipidiques chargées.

b) détermination de la constante maximum de charge d'une kératine délipidée avec du squalène et du squalène peroxydé.

On extrait des kératines de stratum cornéum de porc et on les charge en squalène et squalène peroxydé comme décrit dans le paragraphe a) précédent.

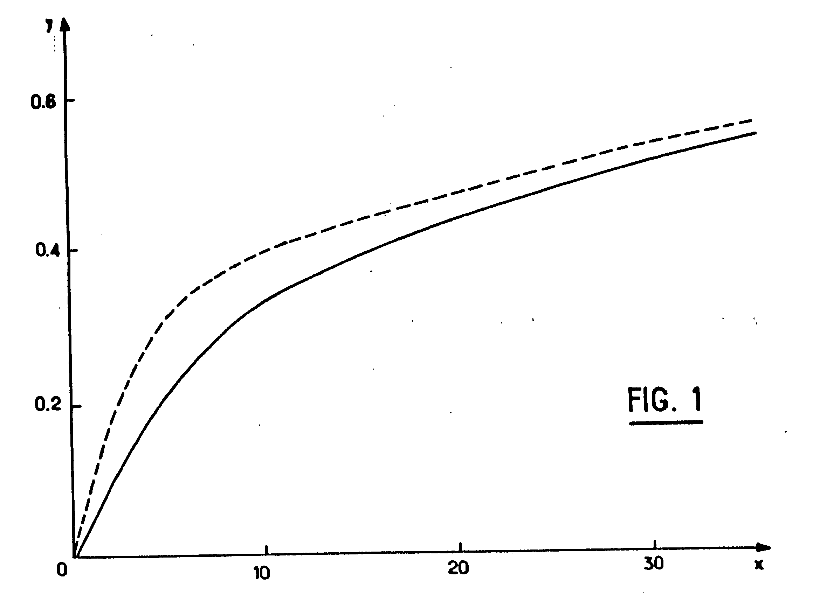

La figure 1 du dessin ci-annexé représente deux courbes, l'une en trait, plein, l'autre en trait pointillé, où l'on a porté en abscisses (x), les charges croissantes de la substance lipidique (respectivement, squalène non peroxydé et squalène peroxydé) exprimées en mg, et, en ordonnées (y), la quantité de squalène (respectivement non peroxydé et peroxydé) rapportée par unité de kératine. On peut observer pour les faibles charges, une vitesse d'association plus rapide—ce qui est indiqué par les pentes à l'origine—pour le squalène peroxydé que pour le squalène non peroxydé, ce qui laisse présager une affinité supérieure de la kératine délipidée pour la forme peroxydée du squalène.

Les conditions pratiques de mise en oeuvre de la kératine délipidée constituant le médicament conforme à la présente invention sont illustrées par la portion des courbes de la figure 1 correspondant sensiblement à une valeur en abscisse comprise entre 0 et 10 mg.

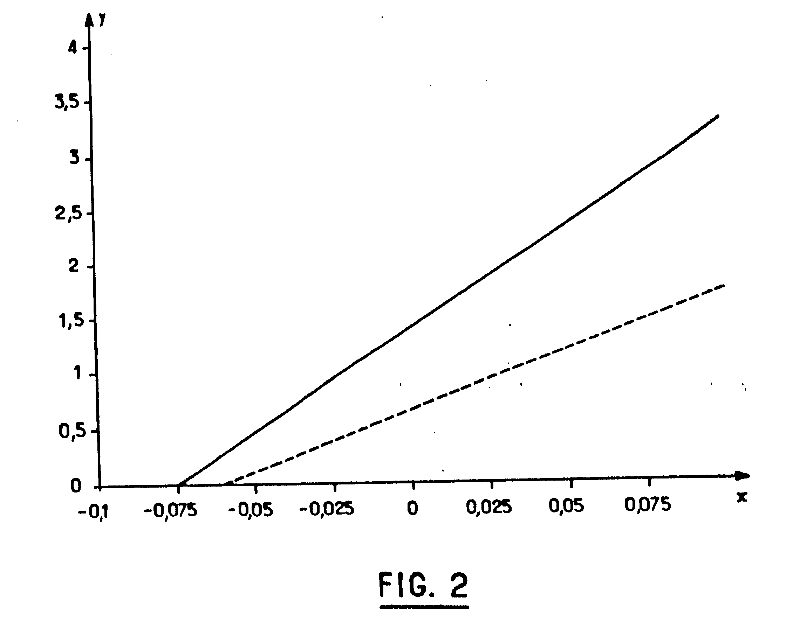

La quantité de charge maximale à saturation est mieux appréhendée par la représentation en inverse de la figure 2, à savoir, le rapport kératine/lipide (non peroxydé et peroxydé) en fonction de l'inverse de la quantité du lipide considéré (soit des valeurs en abscisses exprimées en mg⁻¹ et portées dans l'ordre croissant). La droite obtenue dans le cas d'un chargement en squalène (droite représentée en trait plein sur la figure 2) à pour équation: $y = 18{,}7 \times + 1{,}4$, et celle obtenue dans le cas d'un chargement en squalène peroxydé (droite représentée en trait pointillé sur la figure 2) a pour équation $y = 10{,}83 \times + 0{,}65$.

Le point d'intersection de ces droites de régression avec l'axe des abscisses donne directement les valeurs inverses des constantes maxima de saturation des kératines pour la substance lipidique respectivement non peroxydée et peroxydée, c'est-à-dire la valeur $1/K_s$. Cette valeur est de 0,075 dans le cas du squalène et de 0,060 dans le cas du squalène peroxydé. On constate donc que la constante maximum de charge est plus favorable dans le cas du squalène peroxydé que dans le cas du squalène non peroxydé.

c) détermination des constantes d'affinité pour le squalène peroxydé ou non par représentation en accord avec le modèle de Scatchard

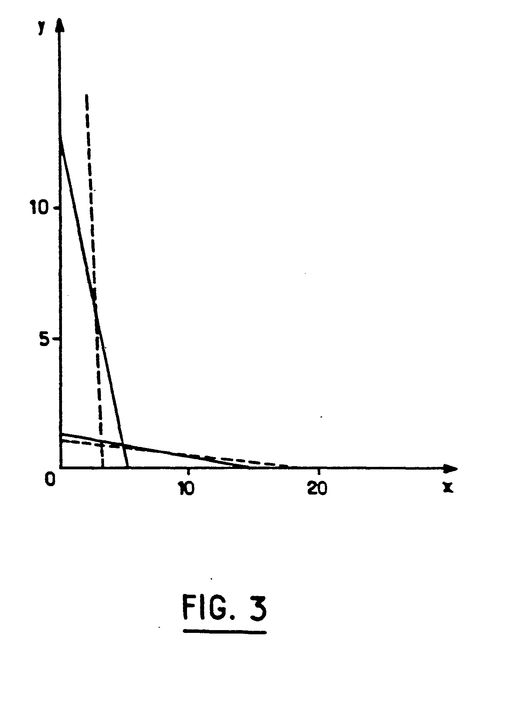

On porte en abscisses (x), la quantité, exprimée en mg de squalène lié, respectivement non peroxydé et peroxydé, et, on ordonnées (y), le rapport du squalène lié (respectivement non peroxydé et peroxydé) sur le squalène libre (respectivement non peroxydé et peroxydé), ce qui permet d'obtenir deux courbes asymptotiques en accord avec la représentation de Scatchard. Sur la figure 3, on a représenté, dans un but de simplification, en trait plein, les trangentes à la courbe obtenue dans le cas du squalène et, en trait pointillé, les tangentes à la courbe obtenue dans le cas du squalène peroxydé. A partir de la pente de chacune de ces tangentes, on déduit les constantes d'affinité qui sont de 2,2 dans le cas du squalène et de 10 dans le cas du squalène peroxydé. Il est remarquable de constater que l'affinité des kératines pouc le squalène peroxydé est près de cinq fois plus élevée que pour le squalène non peroxydé. Ces résultats sont en accord avec ceux obtenus pour la constante maximum de charge (paragraphe b).

d) développement à d'autres lipides peroxydables:

Les mêmes procédés de formation du complexe et d'analyse mathématique ont été appliqués à d'autres lipides également peroxydables: l'acide linoléique, l'acide γ-linolénique et l'acide arachidonique. On donne dans le tableau ci-dessous les résultats obtenus.

TABLEAU

|  | $1/K_s$ |
|---|---|
| Acide linoléique | 0,025 |
| Acide linoléique peroxydé | 0,015 |
| Acide γ-linolénique | 0,020 |
| Acide γ-linolénique peroxydé | 0,010 |
| Acide arachidonique | 0,060 |
| Acide arachidonique peroxydé | 0,045 |

Ce résultat indique la plus grande capacité de charge de ces lipides lorsqu'ils sont sous forme peroxydée.

Par ailleurs, on a noté également une affinité bien supérieure dans le cas de la forme peroxydée par rapport à la forme non peroxydée. Ainsi, dans le cas de l'acide linoléique, les constantes d'affinité, calculées comme indiqué au paragraphe c) ci-dessus, sont de 1,3 pour l'acide linoléique non peroxydé et de 11,9 pour l'acide linoléique peroxydé. Il en résulte que l'affinité des kératines est neuf fois supérieure pour la forme peroxydée par rapport à la forme non peroxydée de l'acide linoléique.

On peut, à partir des quatre exemples étudiés ci-dessus, conclure à une généralisation du rôle de la kératine délipidée comme piégeur des lipides peroxydés.

B) Mise en evidence de l'effet protecteur vis-à-vis de la peroxydation des lipides

a) démonstration in vitro

On expose, pendant 3 heures, à un rayonnement U.V., à raison de 0,353 mW/cm², d'une part, un lot A d'une solution de 20 mg de squalène dans 20 ml d'un mélange chlorofmre/méthanol 2/1 et, d'autre part, un lot B d'une même solution de squalène en présence de 20 mg de kératine délipidée, chaque lot étant agité pendant l'exposition.

A la fin de celle-ci, on a procédé au dosage des peroxydes par la diphénylcarbonohydrazide. Le dosage a montré qu'il s'était formé 1,4 mg de squalène peroxydé dans le cas du lot A et 0,4 mg de squalène peroxydé dans le cas du lot B. Autrement dit, il se forme 3,5 fois moins de peroxyde lorsque la kératine est présente dans le milieu réactionnel, ce qui démontre l'effet protecteur, par la kératine délipidée, vis-à-vis de la peroxydation du squalène.

b) démonstration in vivo

L'expérimentation décrite ci-après est adaptée de celle décrite par W. L. Morison et al, dans l'article intitulé "Variations in the Erythemal Response of Guinea-pig Skin", dans "Photo-Chemistry and Photobiology, Vol. 33, pages 283 à 285", 1981.

Les kératines délipidées utilisées sont des kératines de stratum cornéum de porc à 5% dans une solution d'urée à 0,3% dans l'eau.

Douze cobayes albinos reçoivent, par application, 0,2 ml de kératine sur une zone dorsale, préalablement épilée, d'une surface délimitée par un cercle de 2 cm de diamètre. Douze animaux témoins reçoivent dans les mêmes temps une solution placébo sans kératine, Après séchage, tous les animaux reçoivent soit 1 soit 2 doses minimales érythémateuses (M.E.D.—Minimal Erythem Dose) sous lampe Xénon, c'est-à-dire qu'ils sont exposés au rayonnement donné par cette lampe respectivement pendant un temps suffisant pour faire apparaître un érythème minimum, soit pendant un temps double de ce dernier.

Ces doses érythémateuses étant appliquées, l'intensité de l'érythème est déterminée par la méthode des scores. On obtient après traitement mathématique des données, les résultats groupés dans le tableau suivant:

## TABLEAU

|  | Moyenne±erreur type | Significativité à 0,5%[*] |
|---|---|---|
| 1 MED | Témoin 2±0,27<br>Kératines 0,75±0,14 | protection significative |
| 2 MED | Témoin 2,75±0,28<br>Kératines 1,5±0,22 | protection significative |
| [*] D'après test de Wilcoxon (données non paramétriques). | | |

On a ainsi démontré que les kératines ont protégé in vivo les cobayes soumis au rayonnement U.V. d'une lampe au Xénon.

L'ensemble des expériences précitées a permis de mettre en évidence l'effet des kératines de stratum cornéum (forme délipidée) vis-à-vis 1) des peroxydes déjà formés, qui sont piégés par lesdites kératines, 2) de la formation in vitro des peroxydes, formation qui est inhibée par lesdites kératines, et 3) de l'apparition de l'érythème sous rayonnement U.V., par rapport auquel les kératines assurent un rôle protecteur.

C) Mise en evidence de l'action curative et preventive de la keratine delipidee

L'expérimentation décrite ci-après est le test de comédogénicité sur oreille de lapin, décrit par KLIGMAN AM, KWONG T dans l'article intitulé "An improved rabbit ear model for assessing comedogenic substances" dans "British Journal of Dermatology", vol. 100, p. 99, 1979.

Le protocole d'induction des comédons utilise des lapins albinos mâles dont une oreille est traitée, l'autre servant de témoins. Les oreilles reçoivent une application quotidienne pendant deux semaines (cinq jours sur sept—dix applications au total), à raison de 0,1 ml de chacun des produits par oreille et par jour. Le produit comédogène de référence est ici le squalène peroxydé. Les kératines délipidées utilisées sont des kératines de stratum cornéum de porc à 5% dans une solution d'urée à 0,3% dans l'eau, Le squalène peroxydé et le squalène non peroxydé sont appliqués tels quels. 24 heures après la dernière application, l'épithélium est, prélevé pour observation directe au stéréomicroscope.

L'échelle et le barême de notation sont donnés dans le tableau suivant:

| Pas d'hyperkératose folliculaire | 0 | Non comédogène |
|---|---|---|
| Hyperkératose bien visible-présence possible de comédons | 1 | Légèrement comédogène |
| Comédons bien visibles | 2 | Moyennement comédogène |
| Comédons de grosse taille, nombreux | 3 | Fortement comédogène |

L'expérimentation comporte quatre groupes de cinq animaux chacun, traités comme indiqué dans le tableau ci-après, l'expérimentation sur le groupe I étant effectuée dans le but de contrôler l'absence d'activité comédogène de la kératine, celle sur le groupe II, de mettre en évidence une action préventive de la kératine délipidée, celle sur le groupe III, de mettre en évidence une action curative de la kératine délipidée, et celle sur le groupe IV, de contrôler l'activité comédogène du squalène peroxydé.

6

| Groupe | Oreille traitée recevant: | Oreille témoin recevant: |
|---|---|---|
| I | kératine | eau distillée |
| II | kératine+squalène peroxydé (appliqué deux heures après la kératine) | kératine+squalène non oxydé (appliqué deux heures après la kératine) |
| III | squalène peroxydé+kératine (appliquée deux heures après le squalène oxydé) | squalène non oxydé+kératine (appliquée deux heures après le squalène non oxydé) |
| IV | squalène peroxydé | squalène non oxydé |

Les résultats obtenus sont consignés dans le tableau suivant:

| Groupes | Produits reçus | Effectif noté | | | | Moyenne |
|---|---|---|---|---|---|---|
| | | 0 | 1 | 2 | 3 | |
| I | Eau | 5 | | | | 0 |
| | Kératine | 5 | | | | 0 |
| II | Kératine+squalène non oxydé | 3 | 2 | | | 0,4 |
| | Kératine+squalène peroxydé | | 5 | | | 1 |
| III | Squalène non oxydé+ Kératine | 3 | 2 | | | 0,4 |
| | Squalène peroxydé+ Kératine | | 3 | 2 | | 1,4 |
| | Squalène non oxydé Squalène peroxydé | 5 | | 4 | 1 | 0 2,2 |

On peut donc faire les observations suivantes:

—la kératine et le squalène non oxydé ne sont pas comédogènes, alors que le squalène peroxydé est comédogène (2,2);

—l'association kératine/squalène non oxydé peut être considérée comme non comédogène (0,4);

—la kératine appliquée deux heures avant le squalène abaisse sensiblement l'effet comédogène du squalène peroxydé (2,2→1).

—la kératine appliquée deux heures après le squalène abaisse également l'effet comédogène du squalène peroxydé (2,2→1,4).

En conclusion, les kératines délipidées abaissent l'action comédogène du squalène peroxydé en traitement préventif comme en traitement curatif.

La présente invention a également pour objet une composition médicamenteuse pour le traitement et/ ou la prévention, par application topique, des maladies de la peau mettant en jeu en processus inflammatoire, caractérisée par le fait qu'elle renferme, à titre de principe actif, dans un support pharmaceutiquement acceptable, au moins un médicament tel que défini ci-dessus.

Dans un mode préféré de réalisation, la composition médicamenteuse selon l'invention contient d'environ 1 à environ 10% en poids de principe actif par rappot au poids total de la composition.

De préférence, le support pharmaceutiquement acceptable st constitué par de l'eau; il peut toutefois être constitué par un solvant ou une phase grasse.

La composition selon l'invention peut également renfermer un agent favorisant le gonflement du (ou des) polymère(s) kératinique(s), à raison de 0,3 à 2% en poids par rapport au poids total de la composition.

De préférence, l'agent favorisant le gonflement du (ou des) polymère(s) kératinique(s) est l'urée.

7

La composition selon l'invention peut renfermer, en outre, au moins un adjuvant usuel pris dans le groupe formé par les agents de pénétration, les agents conservateurs, les épaississants.

La composition selon l'invention peut se présenter sous la forme d'une solution, d'une suspension, d'une émulsion ou d'un gel.

Il a été déterminé que l'action thérapeutique recherchée est obtenue avec utilisation de 0,5 à 3 mg de principe actif par cm² de surface en application topique. Le temps d'action est variable. On peut indiquer, que lorsque l'on souhaite désintoxiquer le substrat cutané en éliminant les peroxydes lipidiques déjà formés, on peut laisser agir le médicament pendant une période de temps allant de 15 minutes à 18 heures. Il faut ensuite nettoyer la peau pour éliminer le complexe kératine/peroxyde lipidique. Dans le cas où l'on désire soigner un érythème solaire, on applique aussitôt la composition médicamenteuse et on renouvelle l'application autant de fois que nécessaire. Dans le cas où l'on veut empêcher l'apparition d'un érythème, on applique la composition médicamenteuse avant l'exposition, et on renouvelle éventuellement l'application pendant et après l'exposition.

Exemple 1

On prépare la composition sous forme de gel pour être utilisée en application topique dans le traitement de l'acné, cette composition étant formulée comme suit:

| | |
|---|---|
| —Kératine délipidée obtenue à la fin de la 2ème étape de l'exemple de préparation précédemment décrit | 5 g |
| —Propylèneglycol | 30 g |
| —Hydroxypropylcellulose | 2 g |
| —Eau q.s.p. | 100 g |

Lorsque cette composition a été appliquée régulièrement matin et soir pendant 15 jours sur un patient présentant une peau acnéique, on a observé une régression du développement et du nombre des comédons.

Exemple 2

On prépare une émulsion huile-dans-eau ayant la formulation suivante:

| | |
|---|---|
| —Kératine délipidée obtenue à la fin de la 2ème étape de l'exemple de préparation précédemment décrit | 2 g |
| —Alcool cétylique oxyéthyléné à 20 moles d'oxyde d'éthylène | 0,27 g |
| —Alcool stéarylique oxyéthyléné à 20 moles d'oxyde d'éthylène | 0,63 g |
| —Alcool cétylique | 1,63 g |
| —Alcool stéarylique | 1,47 g |
| —Monostéarate de glycérol | 1 g |
| —Huile de vaseline | 10 g |
| —Eau. . .q.s.p | 100 g |

Cette émulsion a été appliquée sur la peau d'un sujet acnéique ayant subi un traitement, anti-acné à la vitamine A acide. On a observé qu'il a pu s'exposer au soleil sans que l'on ait noté une irritation de la peau.

Cette émulsion a par ailleurs été appliquée sur un sujet souffrant d'érythème solaire. Après quelques heures et deux applications, on a constaté que l'intensité et la surface de l'érythème avaient nettement régressé.

**Revendications**

1. Médicament, utile notamment par son effet complexant sélectif vis-à-vis des lipides présents sur la peau à l'état (per)oxydé et/ou par son effet protecteur vis-à-vis de la (per)oxydation de lipides présents sur la peau et/ou par son effet préventif d'érythèmes, et permettant, en particulier de traiter et/ou de prévenir, par application topique, les maladies de la peau mettant en jeu un processus inflammatoire, ledit médicament consistant en au moins un matériau kératinique obtenu par séparation des substances kératiniques intracellulaires d'une matière première kératinique d'origine animale avec élimination des parois cellulaires, caractérisé par le fait que ledit matériau kératinique est au moins partiellement délipidé.

2. Médicament selon la revendication 1, caractérisé par le fait que le matériau kératinique est totalement délipidé.

3. Médicament selon l'une des revendications 1 ou 2, caractérisé par le fait que la matière première d'origine animale subit une hydrolyse avant délipidation.

4. Médicament selon l'une des revendications 1 ou 2, caractérisé par le fait que la matière première d'origine animale utilisée a subi une hydrolyse après délipidation.

5. Médicament selon l'une des revendications 1 à 4, caractérisé par le fait que la matière première d'origine animale est choisie dans le groupe formé par la corne de sabot, notamment de cheval, les museaux et naseaux, notamment de bovins, la peau animale, notamment de porc, les cheveux et les plumes.

6. Composition médicamenteuse pour le traitement et/ou la prévention par application topique, des maladies de la peau mettant en jeu un processus inflammatoire, caractérisée par le fait qu'elle renferme, à titre de principe actif, dans un support pharmaceutiquement acceptable, au moins un médicament tel que défini à l'une des revendications 1 à 5.

7. Composition selon la revendication 6, caractérisée par le fait qu'elle contient d'environ 1 à environ 10% en poids de principe actif par rapport au poids total de la composition.

8. Composition selon l'une des revendications 6 ou 7, caractérisée par le fait que le support pharmaceutiquement acceptable est constitué par de l'eau, un solvant ou une phase grasse.

9. Composition selon l'une des revendications 6 à 8, caractérisée par le fait qu'elle renferme un agent favorisant le gonflement du (ou des) polymère(s) kératinique(s) à raison de 0,3 à 2% en poids par rapport au poids total de la composition.

10. Composition selon la revendication 9, caractérisée par le fait que l'agent favorisant le gonflement du (ou des) polymère(s) kératinique(s) est l'urée.

11. Composition selon l'une des revendications 6 à 10, caractérisée par le fait qu'elle renferme au moins un adjuvant usuel pris dans le groupe formé par les agents de pénétration, les agents conservateurs et les épaississants.

12. Composition selon l'une des revendications 6 à 11, caractérisée par le fait qu'elle se présente sous la forme d'une solution, d'une suspension, d'une émulsion ou d'un gel.

**Patentansprüche**

1. Medikament, das vor allem aufgrund seiner selektiven, komplexbildenden Wirkung auf Lipide, die auf der Haut in (per)oxidierter Form vorhanden sind, und/oder aufgrund seiner Schutzwirkung von auf der Haut vorhandenen Lipiden vor (Per)oxidation, und/oder aufgrund seiner vorbeugenden Wirkung gegen Erytheme verwendbar ist, und das durch topische Applikation insbesonders eine Behandlung und/oder Prävention von Hautkrankheiten, die durch einen entzündlichen Prozeß verursacht wurden, ermöglicht, wobei das Medikament aus mindestens einer Keratinsubstanz besteht, die man durch Trennung intrazellulärer Keratinsubstanzen von einem Keratinrohstoff tierischen Ursprungs und Beseitigung der Zellwände erhält, dadurch gekennzeichnet, daß die Keratinsubstanz mindestens teilweise von Lipiden befreit ist.

2. Medikament nach Anspruch 1, dadurch gekennzeichnet, daß die Keratinsubstanz vollkommen von Lipiden befreit ist.

3. Medikament nach einem der Ansprüche 1 oder 2, dadurch gekennzeichnet, daß der Rohstoff tierischen Ursprungs vor der Befreiung von Lipiden einer Hydrolyse unterworfen wird.

4. Medikament nach einem der Ansprüche 1 oder 2, dadurch gekennzeichnet, daß der verwendete Rohstoff tierischen Ursprungs nach der Befreiung von Lipiden einer Hydrolyse unterworfen wird.

5. Medikament nach einem der Ansprüche 1 bis 4, dadurch gekennzeichnet, daß der Rohstoff tierischen Ursprungs ausgewählt ist unter dem Horn von Hufen, insbesondere von Pferdem, Mäulern und Nüstern, insbesondere von Rindern, tierischer Haut, insbesondere von Schweinen, Haaren und Federn.

6. Medikamentzusammensetzung zur topischen Behandlung und/oder Prävention von durch einen entzündlichen Prozeß verursachten Hautkrankheiten, dadurch gekennzeichnet, daß sie in einem pharmazeutisch verträglichen Träger als Wirkstoff mindestens ein Medikament nach einem der Ansprüche 1 bis 5 enthält.

7. Zusammensetzung nach Anspruch 6, dadurch gekennzeichnet, daß sie ca. 1 bis ca. 10 Gew.-% des Wirkstoffes, bezogen auf das Gesamtgewicht der Zusammensetzung, enthält.

8. Zusammensetzung nach einem der Ansprüche 6 oder 7, dadurch gekennzeichnet, daß der pharmazeutisch verträgliche Träger Wasser, ein Lösungsmittel oder eine Fettphase ist.

9. Zusammensetzung nach einem der Ansprüche 6 bis 8, dadurch gekennzeichnet, daß sie ein Mittel enthält, das die Quellung des (oder der) Keratinpolymers (Keratinpolymere) begünstigt und das in einer Menge von 0,3 bis 2 Gew.-%, bezogen auf das Gesamtgewicht der Zusammensetzung, vorhanden ist.

10. Zusammensetzung nach Anspruch 9, dadurch gekennzeichnet, daß das Mittel, das die Quellung des (oder der) Keratinpolymers (Keratinpolymere) begünstigt, Harnstoff ist.

11. Zusammensetzung nach einem der Ansprüche 6 bis 10, dadurch gekennzeichnet, daß sie mindestens ein herkommliches Adjuvanz enthält, ausgewählt unter Penetrationsmitteln, Konservierungsmitteln und Verdickungsmitteln.

12. Zusammensetzung nach einem der Ansprüche 6 bis 11, dadurch gekennzeichnet, daß sie in Form einer Lösung, einer Suspension, einer Emulsion oder als Gel vorliegt.

## EP 0 147 261 B1

**Claims**

1. Drug which is especially useful as a result of its selective complexing effect in regard to the lipids present in the (per)oxidized state on the skin and/or as a result of its protective effect in regard to the (per)oxidation of lipids present on the skin and/or as a result of its effect in preventing erythemas, and which enables, in particular, the skin diseases involving an inflammatory process to be treated and/or prevented by topical application, the said drug consisting of at least one keratinous material obtained by separation of the intracellular keratinous substances from a keratinous starting material of animal origin with removal of the cell walls, characterized in that the said keratinous material is at least partially delipidized.

2. Drug according to Claim 1, characterized in that the keratinous material is completely delipidized.

3. Drug according to one of Claims 1 or 2, characterized in that the starting material of animal origin undergoes hydrolysis before delipidation.

4. Drug according to one of Claims 1 or 2, characterized in that the starting material of animal origin used has undergone hydrolysis after delipidation.

5. Drug according to one of Claims 1 to 4, characterized in that the starting material of animal origin is chosen from the group composed of hoof horn, especially of horses, snouts and nostrils, especially of bovines, animal hide, especially of pigs, and hairs and feathers.

6. Drug composition for treating and/or preventing, by topical application, skin diseases involving an inflammatory process, characterized in that it contains, by way of active principle, at least one drug as defined in one of Claims 1 to 5, in a pharmaceutically acceptable carrier.

7. Composition according to Claim 6, characterized in that it contains approximately 1 to approximately 10% by weight of active principle relative to the total weight of the composition.

8. Composition according to one of Claims 6 or 7, characterized in that the pharmaceutically acceptable carrier consists of water, a solvent or a fatty phase.

9. Composition according to one of Claims 6 to 8, characterized in that it contains an agent which promotes swelling of the keratin polymer or polymers, in the proportion of 0.3 to 2% by weight relative to the total weight of the composition.

10. Composition according to Claim 9, characterized in that the agent which promotes swelling of the keratin polymer or polymers is urea.

11. Composition according to one of Claims 6 to 10, characterized in that it contains at least one common adjuvant taken from the group composed of penetrants, preservatives and thickeners.

12. Composition according to one of Claims 6 to 11, characterized in that it takes the form of a solution, a suspension, an emulsion or a gel.

FIG. 1

EP 0 147 261 B1

FIG. 2

FIG. 3